# EUROPEAN PATENT SPECIFICATION

(11) **EP 1 362 837 B1**
(45) Date of publication and mention of the grant of the patent: **30.07.2014**
(21) Application number: 02711407.3
(22) Date of filing: 08.02.2002
(51) Int. Cl.: C07C 2/10, C07C 11/02

(54) **PROCESS FOR PRODUCING LOW POLYMER OF ALPHA-OLEFIN**
VERFAHREN ZUR PRODUKTION EINES NIEDRIGEN POLYMERS VON ALPHA-OLEFIN
PROCEDE DE PRODUCTION D'UN POLYMERE A FAIBLE TENEUR EN ALPHA-OLEFINE

(30) Priority: 23.02.2001 JP 2001048435
(43) Date of publication of application: 19.11.2003
(73) Proprietor: IDEMITSU KOSAN CO., LTD., Tokyo 100-8321 (JP)
(72) Inventor: KOBAYASHI, Ryoichi, Ichihara-shi, Chiba 299-0107 (JP); KURA, Shigeki, Ichihara-shi, Chiba 299-0107 (JP)
(74) Representative: Gille Hrabal
(86) International application number: PCT/JP2002/001074
(87) International publication number: WO 2002/068365

(56) References cited:
- EP-A1- 0 241 596
- JP-A- 8 059 519
- JP-A- 62 000 430
- US-A- 3 108 145
- US-A- 4 377 720
- US-A- 4 740 645

## Description

### TECHNICAL FIELD

The present invention relates to a process for producing an α olefin oligomer which has a double bond and 4 to 24 carbon atoms. More particularly, the present invention is concerned with a process for producing an α -olefin oligomer which has a double bond and 4 to 24 carbon atoms which is capable of producing said α-olefin oligomer in high quality having enhanced purity, by producing an α-olefin oligomer which has a double bond and 4 to 24 carbon atoms and which is useful as a starting material for high molecular polymers, plasticizers, surfactants and the like by the use of a Ziegler based catalyst.

### BACKGROUND ART

An α-olefin oligomer which has a double bond and 4 to 24 carbon atoms is a useful substance which is widely used as a starting monomer material for olefin polymers, as a comonomer for a variety of high molecular polymers, as a starting material for plasticizers, surfactants, etc. The α -olefin oligomer is produced usually by oligomerizing ethylene as the starting raw material by the use of a Ziegler based catalyst. In general, the production process comprises a step of oligomerization reaction, a step of recovering unreacted ethylene, a step of deactivating and deashing the catalyst and a step of fractionating the solvent used therein and the α -olefin oligomer (see, for example, Japanese Patent Application Laid-Open (kokai) No.3-220135).

In the aforesaid production process, the oligomerization reaction is put into practice usually by one step composed of one reactor. In general, the α -olefin oligomer that is produced by the above-mentioned step involves a problem in that it often contains such impurities as paraffin, internal olefin and branched olefin, which bring about marked deterioration in the quality of a polyethylene resin and the like as the final product.

US-4,377,720 discloses a process for producing an alpha-olefin oligomer by subjecting ethylene to a Ziegler based catalyst in a long pipe with introduction of ethylene at many points of this long pipe. No reactors in series are specifically disclosed in this document.

### DISCLOSURE OF THE INVENTION

It has been desired from the above-mentioned standpoint to develop a process for producing an α-olefin oligomer as defined in claim 1 which is capable of producing a high quality α -olefin oligomer minimized in the content of impurities, in the case of subjecting an α -olefin to oligomerization reaction by the use of a Ziegler based catalyst as the origomerization catalyst. The present invention has been made in the light of the foregoing subject.

That is to say, it is a general object of the present invention to provide a process for producing said α-olefin oligomer which is capable of producing said α-olefin oligomer in high quality free from an impurity by the use of a Ziegler based catalyst. In view of the above-mentioned subject, intensive extensive research and investigation were accumulated by the present inventors. As a result, it has been found that the object of the present invention can be achieved by carrying out oligomerization reaction through two stage reaction, supplying each of the two stages with an α-olefin as a starting material component, and controlling the reaction. Thus the present invention has been accomplished on the basis of the foregoing findings and information.

Specifically, the present invention provides a process for producing an α -olefin oligomer as defined in claim 1 which comprises subjecting an α -olefin to oligomerization reaction in an organic solvent in the presence of a Ziegler based catalyst, wherein the foregoing oligomerization reaction is carried out through two reactors arranged in series according to claim 1.

### BRIEF DESCRIPTION OF THE DRAWINGS

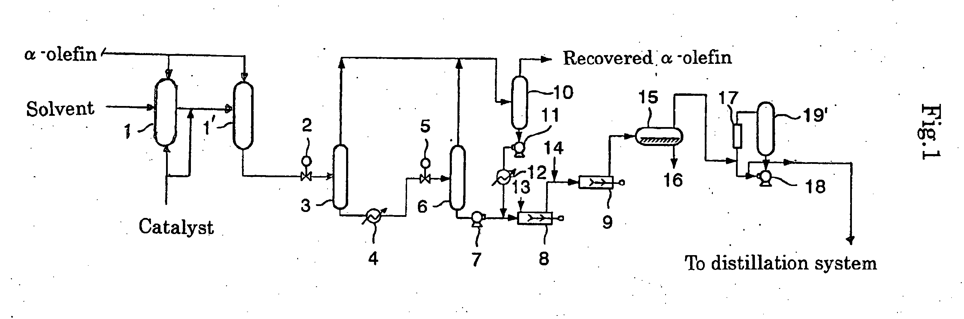

FIG. 1 is an example of schematic process flow diagram which shows the production process for carrying out the present invention.

### THE MOST PREFERRED EMBODIMENT TO CARRY OUT THE INVENTION

In the following, more detailed description will be given of the present invention.

In the present invention, the α-olefin oligomer as defined in claim 1 is obtained by oligomerizing an α-olefin in the presence of a Ziegler based catalyst, which consists of the combination of (A) a transition metal compound, (B) an organoaluminum and (C) a tertiary component to be used as desired. There is used as the transition metal compound (A), the compound represented by the general formula:

M X x Y y O z (I)

wherein M is a zirconium atom or a titanium atom, X is a halogen atom (chlorine atom, bromine atom or iodine atom), Y is RO-, R₂N-, -OCOR, -OSO₃R, R-, -Cp (cyclopentadienyl), wherein R is a straight chain or branched chain alkyl group having 1 to 20 carbon atoms, or β diketonato represented by the general formula: 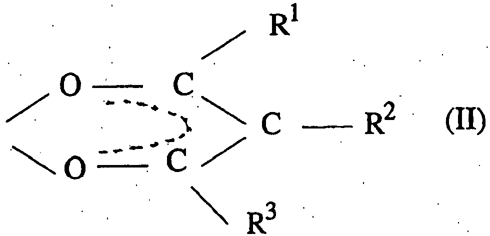 wherein R¹ ,R² and R ³ are each independently a hydrogen atom, an alkyl group having 1 to 20 carbon atoms or an alkyl group which is substituted with a halogen atom and which has 1 to 20 carbon atoms with the proviso that one of R¹, R² and R³ is an alkyl group which is substituted with a halogen atom and which has 1 to 20 carbon atoms, x, y and z are each an integer from 0 to 4 with the proviso that x+ y+ z= 4.

The above-mentioned compound is specifically exemplified by ZrCl₄, ZrBr₄, ZrI₄, ZrBrCl₃, ZrBr₂Cl₂, TiCl₄, TiBr₄, TiI ₄, TiBrCl₃, TiBr₂Cl₂, Zr(OC₂H₅)₄, Zr(OC₂H₅)₂Cl₂, Zr(O-n-C₃H₇)₄, Zr(O-n-C₃H₇)₂Cl₂, Zr(O-iso-C₃H₇)₄, Zr(O-iso-C ₃H₇)₂Cl₂, Zr(O-n-C₄H₉)₄, Zr(O-n-C₄H₉)₂Cl₂, Zr(O-iso-C₄H₉)₄, Zr(O-iso-C₄H₉)₂Cl₂, Zr(O-tert-C₄H₉)₄, Zr(O-tert-C₄H₉)₂Cl₂, Zr((CH₃)₂N)₄, Zr((C₂H₅)₂- N)₄, Zr((n-C₃H₇)₂N)₄, Zr((iso-C₃H₇)₂ N)₄, Zr(n-C₄H₉)₂N)₄, Zr((tert-C₄H₉)₂N)₄, Zr(OSO₃CH₃)₄, Zr(OSO ₃C₂H₅)₄, Zr(OSO₃C₃H₇)₄, Zr(OSO₃C₄H₉)₄, ZrCp₂Cl₂, ZrCp₂ClBr, Ti(OC₂H₅)₄, Tï(OC₂H₅)₂Cl₂, Ti(O-n-C₃H₇)₄, Ti(O-n-C₃H₇Cl₂, Ti(O-iso-C₃H₇)₄, Tï(O-iso-C₃H₇)₂Cl₂, Ti(O-n-C₄H₉)₄, Ti(O-n-C ₄H₉)₂Cl₂, Ti(O-iso-C₄H₉)₄, Ti(O-iso-C₄H₉)₂Cl₂, Ti(O-tert-C ₄H₉)₄, Ti(O-tert-C₄H₉) ₂Cl₂, Ti((CH3) ₂N)₄, Ti((C₂H₅)₂- N)₄, Ti((n-C₃H₇)₂N)₄, Ti((iso-C ₃H₇)₂N)₄,Ti((n-C₄H₉)₂-N)₄, Ti((tert-C₄H₉)₂N)₄, Ti(OSO₃CH₃)₄, Ti(OSO₃C₂H₅)₄, Ti(OSO₃C₃H ₇)₄, Ti(OSO₃C₄H₉)₄, TiCp₂Cl₂, TiCp₂ClBr, Zr(OCOC₂H₅)₄, Zr(OCOC₂H₅) ₂Cl₂, Zr(OCOC₃H₇)₄, Zr(OCOC₃H₇) ₂Cl₂, Zr(OCOC ₃H₇)₄, Zr(OCOC₃H₇) ₂Cl₂, Zr(OCOC₄H₉)₄, Zr(OCOC₄H₉)₂Cl₂, Ti(OCOC₂H₅)₄, Ti(OCOC₂H₅) ₂Cl₂, Ti(OCOC₃H₇)₄, Ti(OCOC₃H ₇)₂Cl₂, Ti(OCOC₃H₇)₄, TiCOCOC₃H₇)₂Cl₂, Ti(OCOC₄H₉)₄, Ti(OCOC₄H₉)₂Cl₂, ZrCl₂ (HCOCFCOF)₂ and ZrCl₂ (CH₃ COCFCOCH₃)₂.

The organoaluminum (B) is exemplified by the compound represented by the general formula:

Al Ya Xb Oc Nd (III)

wherein X is a halogen atom (chlorine atom, bromine atom or iodine atom), Y is RO-, R₂N-, -OCOR, or R-, wherein R is a straight chain or branched chain alkyl group having 1 to 20 carbon atoms, and a, b, c and d are each an integer from 0 to 3 with the proviso that a + b + c + d = 3, or by the compound represented by the general formula:

Al₂ Ya · Xb · Oc · Nd · (IV)

wherein X is a halogen atom (chlorine atom, bromine atom or iodine atom), Y is RO-, R₂N-, -OCOR, -RCOCR'COR" or R-, wherein R, R' and R" are each a straight chain or branched chain alkyl group having 1 to 20 carbon atoms, and a', b', c' and d' are each an integer from 0 to 6 with the proviso that a' + b' + c' + d'= 6.

Examples of the compound represented by the general formula (III) include Al(CH₃)₃, Al(C₂H₅)₃, Al(C₃H₇)₃, Al(iso-C₃H₇)₃, Al(C ₄H₉)₃, Al(iso-C₄H₉)₃, Al(C₅H₁₁)₃, Al(C₆H₁₃)₃, Al(C₈H₁₇)₃, Al(C ₂H₅)₂Cl, Al(C₂H₅)₂Br, Al(C₂H₅)₂I, Al(C₂H₅)Cl₂, Al(C₂H₅)Br₂, Al(C₂H₅)I₂, AlC₂H₅(OC₂H₅)₂, AlC₂H₅(OC₃H₇) ₂, AlC₂H₅(OC₄H₉)₂, Al(OC₂H₅)₂Cl, Al(OC₃H₇)₂Cl, Al(OC₄H₉)₂Cl, Al(OC₂H₅)Cl₂, Al(OC₃H₇)Cl₂, Al(OC₄H₉)Cl₂, AlC₂H₅(OCOC₂H₅)₂, AlC₂ H₅(OCOC₃H₇)₂, AlC₂H₆(OCOC₄H₉)₂, Al(OCOC₂H₅)₂Cl, Al(OCOC₃ H₇)₂Cl, Al(OCOC₄H₉)₂Cl, Al(OCOC₂H₅)Cl₂,Al(OCOC₃H₇)Cl₂ Al(OCOC₄H₉)Cl₂, Al(C₂H₅)₂OC₂H₅, Al(C₂H₅)₂OC₃H₇, Al(C₂H₅)₂ OC₄H₉, Al(C₂H₅)₂N(C₂H₅)₂, Al(C₂H₅)₂N(C₃H₇)₂ and Al(C₂H₅)₂ N(C₄H₉)₂. Examples of the compound represented by the general formula (IV) include Al₂(CH₃)₃Cl₃, Al₂(CH₃)₃Br₃, Al₂(C₂H₅)₃ Cl₃, Al₂(C₂H₅)₃Br₃, Al₂(C₂H₅)₃I₃, Al₂(C₂H₅)₂BrCl₂, Al₂(C₃H ₇)₃Cl₃, Al₂(iso-C₃H₇)₃Cl₃, Al₂(C₄H₉)₃Cl₃, Al₂(iso-C₄H₉)₃Cl₃, Al₂(C₅H₁₁)₃Cl₃, Al₂(C₈H₁₇)₃ Cl₃, Al₂(C₂H₅)₂(CH₃)Cl₃, Al₂(OC ₂H₅)₃Cl₃, Al₂(OC₃H₇)₃Cl₃, Al₂(OC₄H₉)₃Cl₃, Al₂(OCOC₂H₅)₃Cl₃, Al₂(OCOC₃H₇)₃Cl₃ and Al₂(OCOC₄H₉)₃ Cl₃.

As the tertiary component (C) which is used as desired, there is usable at least one compound selected from sulfur compounds, phosphorus compounds and nitrogen compounds. The tertiary component contributes to enhancing the purity of α -olefin oligomer as the objective product.

The sulfur compound needs only to be an organosulfur compound without specific limitation, and is preferably exemplified by dimethyl sulfide, diethyl sulfide, dipropyl sulfide, dihexyl sulfide, dicyclohexyl sulfide, thioethers such as diphenyl thioether: dialkyl disulfide compounds such as dimethyl disulfide, diethyl disulfide, dipropyl disulfide, dibutyl disulfide, dihexyl disulfide, dicyclohexyl disulfide and ethylmethyl disulfide: thiophenes such as thiophene, 2-methyl- thiophene, 3-methylthiophene, 2,3-dimethylthiophene, 2-ethyl- thiophene and benzothiophene and heterocyclic sulfur compounds such as tetrahydrothiophene and thiopyrane: aromatic sulfur compounds such as diphenyl sulfide, diphenyl disulfide, methylphenyl disulfide, methylphenyl sulfide: thioureas: and sulfides such as methyl sulfide, ethyl sulfide and butyl sulfide.

The phosphorus compound needs only to be an organophosphorus compound without specific limitation, and is preferably exemplified by phosphines such as triphenylphosphine, triethylphosphine, tributylphosphine, tripropylphosphine, trioctylphosphine and tricyclohexylphosphine.

The nitrogen compound needs only to be an organonitrogen compound without specific limitation, and is preferably exemplified by organoamines such as methylamine, ethylamine, propylamine, butylamine, pentylamine, hexylamine, cyclohexylamine, octylamine , decylamine, aniline, benzylamine, naphthylamine, dimethylamine, diethylamine, dibutylamine, diphenylamine, methylphenylamine, trimethylamine, triethylamine, tributylamine, triphenylamine, pyridine and picoline.

There are preferably usable in the present invention, the sulfur compounds, phosphorus compounds and nitrogen compounds each as mentioned above, of which is particularly preferably usable one or two or more compounds selected from dimethyl disulfide, thiophenes, thiourea, triphenylphosphine, tributyl phosphine, trioctylphosphine and aniline.

The oligomerization reaction of α -olefin according to the present invention is put into practice usually in an organic solvent. Examples of the organic solvent include naphthene base paraffin such as cyclohexane and decalin, aromatic hydrocarbons such as benzene, toluene, xylene, chlorobenzene, ethylbenzene, dichlorobenzene and chlorotoluene, halogenide thereof, aliphatic hydrocarbons such as pentane, hexane, heptane, octane, nonane and decane, haloalkanes such as dichloroethane and dichlorobutane, and the like solvents.

With regard to the blending proportions of the foregoing components (A), (B) and (C) and the foregoing organic solvent in the present invention, the amount of the component (A) is usually 0.01 to 5 mmol, preferably 0.03 to 1 mmol, the amount of the component (B) is usually 0.05 to 15 mmol, preferably 0.06 to 3 mmol, and the amount of the component (C) is usually 0.05 to 20 mmol, preferably 0.1 to 10 mmol in the case of using the above-mentioned sulfur compound, preferably 0.05 to 5 mmol in the case of using the aforesaid nitrogen or phosphorus compound, on the basis of 250 ml of the organic solvent.

In addition, more preferable result is obtainable by setting the blending proportions of the foregoing components (A) and (B) to 1 to 15 expressed in terms of Al / Zr or Ti (molar ratio).

The oligomerization reaction of an α-olefin in the present invention is carried out through two reactors arranged in series. Specifically, the above-mentioned two reaction steps are constituted of two stage reactors arranged in series, and are particularly preferably constituted of two stage reaction steps.

In the oligomerization reaction constituted of the aforesaid two stage reaction steps according to the present invention, it is indispensable to supply each of the reaction steps with an α-olefin as a starting material component, and with respect to the working effect of the present invention the reacting weight in each of the reaction steps is regulated so as to equalize the reacting weight therein as much as possible. In addition, it is also preferable to regulate the retention time or catalyst supply amount in each of the reaction steps so as to attain such reacting weight in each of the reaction steps. Supply of the above-stated catalyst and organic solvent to the second and subsequent steps is not always necessary, but can be conducted at need.

It is possible to put the reaction conditions and the like into practice specifically in the following manner in each step of the two stage reaction steps.

For instance, a first stage reactor is continuously charged at first with a reaction solvent, catalyst and α-olefin, while enabling the reaction temperature to be set on a temperature at which the catalyst exhibits effective activity, and pressure control to be conducted by regulating the α-olefin supply amount. Reaction liquid thus obtained is taken out therefrom, and is introduced in a second stage reactor, while separately supplying the α-olefin to the second stage reactor. In this case, supply of the above-stated catalyst and organic solvent to the second stage reactor is not always necessary, but can be conducted properly and optionally at need. During the reaction, the reaction ratio, i.e. the ratio of the oligomerizing reacting weight in the first stage reactor to that in the second stage reactor, that is, the ratio of (the oligomerizing reacting weight in the first stage reactor): (the oligomerizing reacting weight in the second stage reactor) is preferably in the range of 30 : 70 to 70: 30. When the above-mentioned ratio departs from such range, impurities in the α-olefin oligomer to be obtained often increase, deteriorating the purity of the product. In particular in the present invention, the foregoing ratio is more preferably in the range of 40 : 60 to 60 : 40, particularly preferably 50 : 50, approximately.

In the present invention, the oligomerization reaction in each of the reaction steps of the above-mentioned two stage reaction steps can be carried out usually at a temperature in the range of 100 to 150°C under pressure of 30 to 90 kg / cm² · G (2.94 to 8.82 MPa). The reaction time in each of the reaction steps, which varies depending upon the temperature and pressure and accordingly can not be unequivocally determined, is usually 5 to 40 minutes per each of the steps, making a total of 10 to 60 minutes, approximately.

In the process according to the present invention, the liquid reaction product obtained by the oligomerization reaction of an α -olefin is subjected to subsequent recovery of unreacted α -olefin, deactivation of the catalyst and deashing treatment. In this case, it is preferable to maintain the temperature of the liquid reaction product after the completion of the oligomerization reaction at 90°C or higher. The temperature thereof is not specifically limited provided that it is 90°C or higher, but is in the range of usually 90 to 150°C, preferably 100 to 130°C. The temperature thereof, when being unreasonably high, is unfavorable, since it often brings about deterioration of product purity.

The amount of by-produced polymer, which varies depending upon the reaction conditions, is not unequivocal, but is usually 300 to 500 ppm. The by-produced polymer is dissolved in the liquid reaction product when the temperature thereof is kept at 90°C or higher, thereby enabling to proceed with stable running irrespective of the type of the organic solvent to be used for the oligomerization reaction.

Subsequently the catalyst is subjected to deactivation treatment by introducing a deactivating agent at a pressure of the treatment system of 4 kg / cm ² · G(0.39 MPa), approximately. Examples of the deactivating agent to be used therein include basic nitrogen compounds, water, alcohols, carboxylic acids and phenols. The basic nitrogen compounds among them are exemplified by ammonia and amines such as methylamine, ethylamine, propylamine, butylamine, pentylamine, hexylamine, cyclohexylamine, octylamine, decylamine, aniline, benzylamine, naphthylamine, dimethylamine, diethylamine, dibutylamine, diphenylamine, methylphenylamine, trimethylamine, triethylamine, tributylamine, triphenylamine, pyridine and picoline.

In the present invention, the above-mentioned deactivation treatment is followed by deashing treatment and further recovery of the organic solvent and unreacted α -olefin by distillation. The recovered organic solvent and unreacted α-olefin are each recycled at need through the oligomerization reaction system.

The objective α-olefin oligomers in the present invention are obtained as desirable mixed products of various α-olefin oligomers by means of distillation. The mixed products can be obtained in large amounts as α-olefin oligomers each having desirable number of carbon atoms by properly and optionally selecting the reaction conditions.

In the following, some description will be given of the preferred embodiments of the present invention with reference to the attached drawings. FIG. 1 is an example of schematic process flow diagram which shows the production process for carrying out the present invention by using reactors composed of two stage reaction steps. In the process as illustrated in FIG. 1, a first reactor 1 is charged with a reaction catalyst, a reaction solvent and an α-olefin as a starting material to proceed with oligomerization reaction. Thus the liquid reaction product which is produced therein and composed of the reaction catalyst, the reaction solvent, unreacted α-olefin and α-olefin oligomers is introduced to a second reactor 1', which is further supplied with the α-olefin as the starting material to proceed with oligomerization reaction. Likewise, the liquid reaction product which is produced in the second reactor 1' and composed of the reaction catalyst, the reaction solvent, unreacted α-olefin and α -olefin oligomers is supplied to a first stage flash tank 3 via a control valve 2, and further to a second stage flash tank 6 via a control valve 5. The liquid product after the first stage flashing, prior to supply to the second stage flash tank, is heated in a heat exchanger 4 to be kept at a prescribed temperature or higher. In these flash tanks, the unreacted α -olefin which is dissolved in the liquid reaction product is recovered. Thereafter the liquid reaction product is sent to a deactivator 8, where the catalyst is deactivated with a deactivating agent 13. Thus a slight amount of light α-olefin oligomer accompanying the recovered α-olefin is recovered in a pot 10, and is sent to the deactivator 8, and then to a deasher 9, and after cleaning with cleaning water 14, to a separating tank 15. Therein the α -olefin oligomer is separated into oil phase and water phase, and the water phase is discarded to the outside of the reaction e system as waste water 16. The oil phase is sent to a dissolving tank 19 equipped with a heat exchanger 17 and a pump 18, is again heated to completely dissolve the polymer in the oil phase, and thereafter is sent to the distillation system, where the solvent and the α-olefin are fractionated.

In summarizing the working effect and advantage of the present invention, it is made possible thereby to produce highly pure α-olefin oligomer having a double bond and 4 to 24 carbon atoms free from an impurity in the production of α-olefin oligomer by the used of a Ziegler based catalyst.

In what follows, the present invention will be described in more detail with reference to working examples, which however shall never limit the present invention thereto.

### Example 1

### [Preparation of catalyst]

In a 500 milliliter (mL) flask equipped with a stirrer were introduced in an atmosphere of argon, 25 mmol of zirconium tetrachloride anhydride (ZrCl₄) and 250 mL of dry cyclohexane with stirring for 10 minutes at room temperature. To the mixture thus prepared were added triethylaluminum [(C₂H₅)₃Al] and then ethylaluminum sesquichloride [(C₂H₅)₃Al₂Cl₃], wherein the amounts of the triethylaluminum and ethylaluminum sesquichloride were regulated to (C₂H₅)₃Al₂Cl₃ / (C₂H₅)₃Al being 3.5 (molar ratio) and [(C₂H₅)₃Al₂Cl₃ + (C₂H₅)₃Al] / ZrCl₄ being 7 (molar ratio). After adding all the components, the resultant mixture was heated at 70°C for 2 hours in an atmosphere of argon under stirring to form a complex so that liquid catalyst was prepared.

### [Oligomerization reaction]

Oligomerization reaction was continuously carried out by arranging in series, two sets of complete mixing tank type reactors (internal volume of 500 ml each), taking out reaction liquid from the first stage reactor, and supplying the second stage reactor with the reaction liquid.

The above-prepared liquid catalyst was mixed with cyclohexane which had been dried in an atmosphere of argon so that the concentration of the zirconium tetrachloride was adjusted to 0.08 mmol /1 mmol of cyclohexane. Further, thiophene was added to the mixture in an amount of three times molar ratio to the zirconium tetrachloride to prepare a catalytic solution. Subsequently a definite amount (700 ml/hour) of the catalytic solution was fed in the first stage reactor. The oligomerization reaction was carried out by taking out reaction liquid from the first stage reactor, while regulating the liquid level to a constant value in the first stage reactor, and supplying the second stage reactor with the reaction liquid under the reactional conditions including a reaction temperature of 120°C, reaction pressure of 65 kg /cm² G (6.4 MPa ), stirring at a revolutional speed of 500 rpm, and continuous supply of highly pure ethylene gas so as to maintain the reaction pressure at 65 kg / cm² · G (6.4 MPa) in each of the reactors. The liquid level was equivalent to 200 cc in the first stage reactor and 250 ml in the second stage reactor. The reaction time (retention time) based on the solvent was about 17 minutes in the first stage reactor and about 21 minutes in the second stage reactor. The reaction conditions and the results are collectively given in Table 1.

### [Deactivation treatment of catalyst]

The deactivation treatment of catalyst was carried out by continuously supplying the deactivating tank with the liquid reaction product which had been obtained in the above-mentioned oligomerization reaction. A deactivating agent consisting of 10% by weight of aqueous ammonia was supplied at 28 g / hour. The deactivating tank was operated at 100°C at 4 kg / cm² · G (0.39 MPa) under stirring at a revolutional speed of 700 rpm. The liquid product after the deactivation treatment was filtered to filter off wax component by using filter paper. The resultant filtrate was washed twice with deionized water in an amount two times that of the filtrate, and then was dried with potassium carbonate anhydride. The colorless transparent liquid reaction product thus obtained was analyzed by gas chromatography to determine the distribution and purity of the α -olefin oligomer as the objective product. The product distribution was found by calculation through Schultz · Flory distribution from the result of gas chromatography for C-10 and more based on the operational loss. The results are given in Table 1.

Further in order to determine the reacting weight in the first stage reactor, a sample of the reaction liquid was collected from the first stage reactor, and was analyzed by gas chromatography in the same manner as above to determine the amount of the α -olefin oligomer thus formed.

The reaction ratio in each of the reactors is calculated by [(reacting weight in each reactor / (total reacting weight in the all the reactors)) × 100 (molar percent). In Table 1, C 18 purity is the production ratio of 1-octadecene as the objective product to the total production amount of C 18 components.

### Examples 2 & 3

The procedure in Example 1 was repeated to carry out the oligomerization reaction except that the liquid level in the first stage reactor and also the liquid level in the second stage reactor were altered to the levels as given in Table 1. The reaction conditions and performance results are given in Table 1.

### Example 4

The procedure in Example 3 was repeated to carry out the oligomerization reaction except that the feed lines of the catalyst and solvent leading to the first stage reactor were branched, and the catalyst and solvent in part were directly supplied to the second stage reactor through the branched lines. The reaction conditions and performance results are given in Table 1.

### Example 5 (not according to the invention)

In carrying out the oligomerization reaction in Example 1, a third stage reactor same as the first and second stage reactors was installed on the downstream side of the second stage reactor constituting three-stage reaction steps. Thus the oligomerization reaction was put into practice under the reactional conditions as given in Table 1, wherein the reactional conditions in the third stage reactor were set to a reaction temperature of 120°C, reaction pressure of 65 kg / cm ² · G (6.4 MPa), stirring at a revolutional speed of 500 rpm, and continuous supply of highly pure ethylene gas so as to maintain the reaction pressure at 65 kg / cm² · G (6.4 MPa), which were same as in the first and second stage reactors. The reaction conditions and performance results are given in Table 1.

### Comparative Example 1

The procedure in Example 1 was repeated to carry out the oligomerization reaction except that use was made of a first stage reactor having an internal volume of 1000 ml, the liquid level therein was set on 500 ml, and a second stage reactor was not used. The reaction conditions and performance results are given in Table 1.

**Table 1-1**

| | | Example No. | | |
|---|---|---|---|---|
| | | 1 | 2 | 3 |
| (Supply Amount of Catalyst and Solvent) | | | | |
| (First Stage Reactor) | | | | |
| ZrCl₄ | (mmol/hour) | 0.08 | 0.08 | 0.08 |
| Ethylaluminum sesquichloride | (mmol/hour) | 0.436 | 0.436 | 0.436 |
| Triethylaluminum | (mmol/hour) | 0.124 | 0.124 | 0. 124 |
| Cyclohexane | (ml/hour) | 700 | 700 | 700 |
| (Second Stage Reactor) | | | | |
| ZrCl₄ | (mmol/hour) | 0 | 0 | 0 |
| Ethylaluminum sesquichloride | (mmol/hour) | 0 | 0 | 0 |
| Triethylaluminum | (mmol/hour) | 0 | 0 | 0 |
| Cyclohexane | (ml/hour) | 0 | 0 | 0 |
| <Third Stage Reactor> | | | | |
| ZrCl₄ | (mmol/hour) | - | - | - |
| Ethylaluminum sesquichloride(mmol/hour) | | - | - | - |
| Triethylaluminum | (mmol/hour) | - | - | - |
| Cyclohexane | (ml/hour) | - | - | - |
| <Reaction temperature> | (°C) | 120 | 120 | 120 |
| <Reaction pressure> | (MPa) ((kg / cm² · G)) | 6.4 (65) | 6.4 (65) | 6.4 (65) |

**Table 1-2**

| | | Example No. | | Comp. ExNo. 1 |
|---|---|---|---|---|
| | | 4 | 5 * | |
| (Supply Amount of Catalyst and Solvent) | | | | |
| <First Stage Reactor> | | | | |
| ZrCl₄ | (mmol/hour) | 0.07 | 0.08 | 0.08 |
| Ethylaluminum sesquichloride | (mmol/hour) | 0.374 | 0.436 | 0.436 |
| Triethylaluminum | (mmol/hour) | 0.1 | 0.124 | 0.124 |
| Cyclohexane | (ml/hour) | 600 | 700 | 700 |
| <Second Stage Reactor> | | | | |
| ZrCl₄ | (mmol/hour) | 0.01 | 0 | - |
| Ethylaluminum sesquichloride | (mmol/hour) | 0.062 | 0 | - |
| Triethylaluminum | (mmol/hour) | 0.018 | 0 | - |
| Cyclohexane | (ml/hour) | 100 | 0 | - |
| <Third Stage Reactor> | | | | |
| ZrCl₄ | (mmol/hour) | - | 0 | - |
| Ethylaluminum sesquichloride | (mmol/hour) | - | 0 | - |
| Triethylaluminum | (mmol/hour) | - | 0 | - |
| Cyclohexane | (ml/hour) | - | 0 | - |
| (Reaction temperature) | (°C ) | 120 | 120 | 120 |
| <Reaction pressure> | (MPa) (kg / cm² · G)) | 6.4 (65) | 6.4 (65) | 6.4 (65) |

| | | | | |
|---|---|---|---|---|
| {Remarks} Comp. ExNo. 1: Comparative Example 1 * not according to the invention | | | | |

**Table 1 - 3**

| | | | Example No. | | |
|---|---|---|---|---|---|
| | | | 1 | 2 | 3 |
| <First Stage Reactor> | | | | | |
| Reactor level | | (ml) | 200 | 230 | 250 |
| Reaction time | | (minute) | 17 | 20 | 21 |
| Reacting weight | | (g/hour) | 119 | 138 | 151 |
| Reaction ratio (Proportion) | | (molar% ) | 52 | 60 | 67 |
| <Second Stage Reactor> | | | | | |
| Reactor level | | (ml) | 250 | 230 | 200 |
| Reaction time | | (minute) | 21 | 20 | 17 |
| Reacting weight | | (g/hour) | 110 | 92 | 74 |
| Reaction ratio (Proportion) | | (molar%) | 48 | 40 | 33 |
| <Third Stage Reactor> | | | | | |
| Reactor level | | (ml) | - | - | - |
| Reaction time | | (minute) | - | - | - |
| Reacting weight | | (g/hour) | - | - | - |
| Reaction ratio (Proportion) | | (molar%) | - | - | - |
| Catalytic activity (kg/g • ZrCl₄) | | | 12.8 | 12.9 | 12.6 |
| α -Olefin oligomer thus formed | C 4 | (% by weight) | 15.0 | 14.7 | 14.8 |
| | C 6 | (% by weight) | 15.4 | 15.2 | 15.2 |
| | C 8 | (% by weight) | 14. 1 | 14.0 | 14.0 |
| | C 10 to 16 | (% by weight) | 36.3 | 36.4 | 36.4 |
| | C 18 | (% by weight) | 4.8 | 4.9 | 4.9 |
| | C 20 and more | (% by weight) | 14.4 | 14.8 | 14.7 |
| | C 18 purity | (% by weight) | 96.0 | 95.8 | 95.4 |

**Table 1 - 4**

| | | | Example No. | | Comp. ExNo.1 |
|---|---|---|---|---|---|
| | | | 4 | 5 * | |
| <First Stage Reactor> | | | | | |
| Reactor level | | (cc) | 250 | 100 | 500 |
| Reaction time | | (minute) | 25 | 9 | 43 |
| Reacting weight | | (g/hour) | 121 | 88 | 225 |
| Reaction ratio (Proportion) | | (molar%) | 55 | 38 | 100 |
| <Second Stage Reactor> | | | | | |
| Reactor level | | (ml) | 200 | 150 | - |
| Reaction time | | (minute) | 17 | 13 | - |
| Reacting weight | | (g/hour) | 99 | 81 | - |
| Reaction ratio (Proportion) | | (molar%) | 45 | 35 | - |
| <Third Stage Reactor> | | | | | |
| Reactor level | | (ml) | - | 200 | - |
| Reaction time | | (minute) | - | 17 | - |
| Reacting weight | | (g/hour) | - | 63 | - |
| Reaction ratio (Proportion) | | (molar% ) | - | 27 | - |
| Catalytic activity (kg/g • ZrCl₄) | | | 12.3 | 13.0 | 12.6 |
| α -Olefin oligomer thus formed | C 4 | (% by weight) | 15.2 | 14.6 | 14.9 |
| | C 6 | (% by weight) | 15.6 | 15.1 | 15.4 |
| | C 8 | (% by weight) | 14.2 | 13.9 | 14.1 |
| | C 10 to 16 | (% by weight) | 36.3 | 36.4 | 36.3 |
| | C 18 | (% by weight) | 4.8 | 4.9 | 4.9 |
| | C 20 and more | (% by weight) | 13.9 | 15.1 | 14.4 |
| | C 18 purity | (% by weight) | 95.9 | 96.3 | 94.5 |

| | | | | | |
|---|---|---|---|---|---|
| {Remarks} Comp. ExNo.1: Comparative Example 1 * not according to the invention | | | | | |

### INDUSTRIAL APPLICABILITY

The present invention relates to a process capable of producing a highly pure and high quality α -olefin oligomer having a double bond and 4 to 24 carbon atoms by the use of a Ziegler based catalyst, which is useful as a starting material for high molecular polymers, plasticizers, surfactants and the like.

## Claims

1. A process for producing an α-olefin oligomer which comprises subjecting an α-olefin to oligomerization reaction in an organic solvent in the presence of a Ziegler based catalyst, wherein
(1) said oligomerization reaction is carried out through two reactors arranged in series feeding ethylene to each of the reaction stages;
(2) the reaction ratio of the oligomerizing reaction weight in the first reaction step to the oligomerizing reaction weight in the second reaction step, which is defined as (the oligomerizing reacting weight in the first stage reactor):(the oligomerizing reacting weight in the second stage reactor), is in the range of 30:70 to 70:30; and
(3) an α-olefin oligomer is produced which has a double bond and 4 to 24 carbon atoms.

## Patentansprüche

1. Verfahren zur Herstellung eines α-Olefin-Oligomeren, welches Durchführung einer Oligomerisationsreaktion mit einem α-Olefin in einem organischen Lösungsmittel in Gegenwart eines Katalysators auf Ziegler-Basis umfasst, wobei
(1) die Oligomerisatlonsreaktion durch zwei Reaktoren durchgeführt wird, die in Reihe geschaltet sind, wobei Ethylen in jede der Reaktionsstufen zugeführt wird;
(2) das Reaktionsverhältnis des Oligomerisationsreaktionsgewichts im ersten Reaktionsschritt zum Ollgomerisationsreaktionsgewicht im zweiten Reaktionsschritt, das definiert ist als (Oligomerisatlonsreaktionsgewicht im Reaktor des ersten Schritts) : (Oligomerisatlonsreaktionsgewicht im Reaktor des zweiten Schritts) im Bereich von 30; 70 zu 70:30 ist; und
(3) ein α-Olefin-Oligomer gebildet wird, welches eine Doppelbindung und 4 bis 24 Kohlenstoffatome aufweist.

## Revendications

1. Procédé de production d'un oligomère α-oléfinique qui comprend l'étape consistant à soumettre un α-oléfine à une réaction d'oligomérisation dans un solvant organique en présence d'un catalyseur à base de Ziegler, dans lequel
(1) ladite réaction d'oligmérisation est mise en oeuvre par voie de deux réacteurs arrangés en série alimentant l'éthylène à chacune des étapes de réaction ;
(2) le rapport de réaction du poids de réaction d'oligmérisation dans la première étape de réaction est défini en tant que (le poids de réaction d'oligmérisation dans le réacteur de la première étape) : (le poids de réaction d'oligmérisation dans le réacteur de la deuxième étape) est dans la gamme de 30 :70 à 70 :30 ; et
(3) un oligomère est produit qui a une liaison double et 4 à 24 atomes de carbone.
